# EUROPEAN PATENT APPLICATION

(11) **EP 4 821 496 A1**
(43) Date of publication of application: **14.10.2026**
(21) Application number: 24899903.9
(22) Date of filing: 05.12.2024
(51) Int. Cl.: A61K 31/454, A61K 31/706, C07D 401/02, C07D 401/06, C07D 403/02, C07D 403/06, A61P 13/12

(54) **USE OF BENZO NITROGEN-CONTAINING HETEROAROMATIC RING DERIVATIVE IN TREATING COMPLEMENT FACTOR B-MEDIATED DISEASE**

(30) Priority: 05.12.2023 CN 202311649658; 10.07.2024 CN 202410922608
(71) Applicant: Xizang Haisco Pharmaceutical Co., Ltd., Shannan, Xizang 856099 (CN)
(72) Inventor: GAO, Shidong, Chengdu, Sichuan 611130 (CN); LI, Yangguang, Chengdu, Sichuan 611130 (CN); WANG, Ju, Chengdu, Sichuan 611130 (CN); YANG, Juan, Chengdu, Sichuan 611130 (CN); WANG, Chao, Chengdu, Sichuan 611130 (CN); WU, Qinghe, Chengdu, Sichuan 611130 (CN); LI, Fangqiong, Chengdu, Sichuan 611130 (CN)
(74) Representative: AWA Denmark A/S
(86) International application number: PCT/CN2024/137049
(87) International publication number: WO 2025/119266

(57) **Abstract**

Disclosed is use of a compound of formula (I) or a stereoisomer, a tautomer, a pharmaceutically acceptable salt, an eutectic thereof, or a composition thereof in the preparation of a drug for treating a complement factor B-mediated disease. Also disclosed is a method for treating the complement factor B-mediated disease. The method comprises administering an effective dose of the compound of formula (I) or the stereoisomer, the tautomer, the pharmaceutically acceptable salt, the eutectic thereof, or the composition thereof.

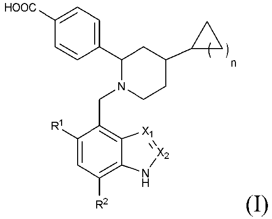

## Description

### Technical Field

The present invention relates to use of a compound of formula (I), or a stereoisomer, tautomer, pharmaceutically acceptable salt, cocrystal or composition thereof in the preparation of a drug for treating a complement factor B-mediated disease.

### Background Art

Dysregulation of complement system homeostasis is associated with a series of renal and hemolytic diseases, including atypical hemolytic uremic syndrome (aHUS), anti-neutrophil cytoplasmic antibody (ANCA)-mediated vasculitis, paroxysmal nocturnal hemoglobinuria (PNH), C3 glomerulopathy (C3G), IgA nephropathy (IgAN), immune complex-mediated membranoproliferative glomerulonephritis, lupus nephritis, membranous nephropathy, chronic transplant glomerulopathy, and the like.

Primary IgA nephropathy is the most common primary glomerulonephritis. China has a high prevalence of IgA nephropathy, which accounts for 45%-50% of primary glomerular diseases. Approximately 80% of patients with IgA nephropathy are young and middle-aged adults. The disease follows a chronic progressive course in the vast majority of patients, and 30%-40% of individuals with IgAN develop end-stage renal disease within 10-20 years after diagnosis. The pathogenesis of IgAN has not yet been fully elucidated. It is currently believed to be associated with the combined effects of mechanisms such as autoimmunity, inflammatory activation, genetic and environmental factors. The pathology is characterized by the deposition of immunoglobulin A (IgA) in the mesangial region of the glomerulus, with or without C3 deposition. C3 nephropathy (C3 Glomerulopathy) is a rare and serious kidney disease that has only been recognized in recent years, with about 60% of C3G entering end-stage renal disease within 10 years of diagnosis. The pathology of C3G is characterized by the deposition of complement C3 along the glomerular capillary loops on immunofluorescence staining, without the deposition of other immunoglobulins. It is a glomerulonephritis characterized by electron-dense deposits in the subendothelial space of glomerular capillary loops and/or the mesangial area under electron microscopy. Pharmacological treatment for primary glomerular diseases mainly comprises immunosuppressive therapy, and angiotensin-converting enzyme inhibitors (ACEI), or angiotensin II receptor blockers (ARB). Kidney diseases involving glomerular damages caused by systemic diseases are secondary glomerular diseases, such as lupus nephritis, renal damage due to systemic vasculitis, and diabetic nephropathy. Treatment for lupus nephritis should be individualized based on clinical and pathological findings. Pharmacological therapies mainly include hormones, immunosuppressants, ACEI/ARB, biological macromolecule monoclonal antibody, and the like. In general, current therapeutic options remain limited and can only partially delay the progression to end-stage renal disease, which fails to meet existing clinical needs.

Paroxysmal nocturnal hemoglobinuria (PNH) is a complement-driven hemolytic anemia. Mutations in the phosphatidylinositol glycan class A (PIG-A) gene on the X chromosome of hematopoietic stem cells lead to impaired biosynthesis of glycosylphosphatidylinositol (GPI). This results in the loss of GPI-anchored proteins on the surface of hematopoietic stem cells and their progeny (red blood cells, granulocytes, monocytes, and lymphocytes), including CD55 and CD59 molecules. The former can inhibit the formation of complement C3 convertase, while the latter can prevent the assembly of soluble complement C9 into a membrane attack complex. Loss of CD55 and CD59 on membranes of red blood cells results in intravascular hemolysis. Patients with PNH may present with hemolytic anemia, thrombophilia, and bone marrow failure. PNH is a rare disease mainly characterized by chronic intravascular hemolysis and bone marrow failure, with an incidence of about 1.3 per million and a prevalence of about 15.9 per million.

Atypical hemolytic uremia (aHUS) is a thrombotic microangiopathy caused by complement disorders, characterized mainly by microangiopathic hemolytic anemia, thrombocytopenia and damage to end-organs (mainly the kidneys). The onset of the disease is mostly during infancy, with an annual incidence of 3.3 per 100,000 children. Among children with hemolytic uremic syndrome, typical HUS accounts for 85%-90%, while aHUS accounts for 5%-10%.

Complement inhibitors that target terminal complement are the preferred treatment methods for PNH and aHUS. In 2007, eculizumab (a monoclonal antibody that blocks the C5 terminal complement) altered the natural course of PNH and aHUS. There are several new complement inhibitors that can inhibit both C5 and complements upstream of C5. To date, however, only eculizumab and ravulizumab have been approved by the U.S. Food and Drug Administration (FDA) and the European Medicines Agency for the treatment of PNH and aHUS.

Eculizumab and ravulizumab are currently the only FDA-approved drugs for the treatment of PNH and aHUS. Thrombosis, severe anemia or PNH symptoms (chronic fatigue, pain, dyspnea) are strong indications for initiating treatment. Terminal complement inhibition will increase the risk of Neisseria infection. Therefore, all patients on eculizumab are recommended to be vaccinated against Neisseria. Even with vaccination, the risk of infection with Neisseria is > 1000 times higher than that of the healthy control group, with an annual absolute risk of up to 0.5%. Gupta et al.'s study found that patients with first pregnancy-associated aHUS had a significantly better prognosis with eculizumab treatment than in women who did not receive eculizumab treatment.

Eculizumab and ravulizumab prevent intravascular hemolysis and thrombosis during the natural course of PNH; however, room for improvement remains. More than 50% of patients with PNH continue to experience mild to moderate PNH symptoms despite treatment with C5 inhibitors, and up to 20% of patients still require occasional blood transfusions. The most common cause of persistent anemia is extravascular hemolysis. Novel complement inhibitors are under development for the treatment of PNH, and some may receive FDA approval within the next one to two years. Pegcetacoplan is a C3 inhibitor administered subcutaneously twice weekly, which can block both intravascular and extravascular hemolysis. It is being compared head-to-head with eculizumab in a phase 3 clinical trial. Danicopan is another promising agent that blocks the complement alternative pathway by inhibiting complement factor D.51. Danicopan is administered orally, and can increase hemoglobin levels and eliminate transfusion requirements in transfusion-dependent PNH patients. In December 2023, the FDA approved iptacopan, a small molecule inhibitor of complement factor B, for the treatment of PNH. This approval was mainly based on the pivotal phase 3 clinical trial of iptacopan in patients with paroxysmal nocturnal hemoglobinuria (PNH), which met two primary endpoints: the proportion of subjects with an increase in hemoglobin level of ≥ 2 g/dL relative to baseline and a stable hemoglobin level of 12 g/dL or higher after week 24. Furthermore, among adult patients with PNH who had previously received complement inhibitor therapy but continued to suffer from anemia, iptacopan was superior to anti-C5 therapies (eculizumab or long-acting ravulizumab), providing a safer and more effective treatment option for patients with PNH. Therefore, the development of novel small molecule inhibitors of complement B can better control both intravascular and extravascular hemolysis, addressing the limitations of anti-C5 antibodies; meanwhile, it provides an oral monotherapy option for patients with PHN and aHUS, holds promise in addressing the unmet clinical needs of complement-related diseases, and has significant clinical and social value.

WO 2023020566 discloses a compound of formula (I), which exhibits good inhibitory activity against complement factor B and a high inhibition rate of in vivo C3a levels,

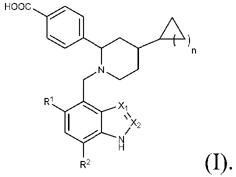

### Summary of the Invention

The present invention provides use of a compound of formula (I), or a stereoisomer, tautomer, pharmaceutically acceptable salt, cocrystal or composition thereof in the preparation of a drug for preventing or treating a complement factor B-mediated disease or condition, 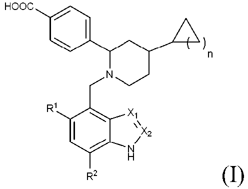 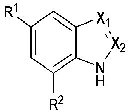 is selected from 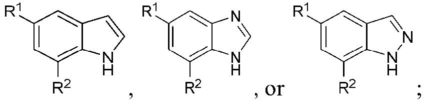
R¹ is selected from -OCH₃ or -OCD₃;
R² is selected from -CH₃ or -CD₃;
n is selected from 1, 2 or 3.

Further, the structure of the compound represented by general formula (I) is selected from one of the structures shown in Table S-1.

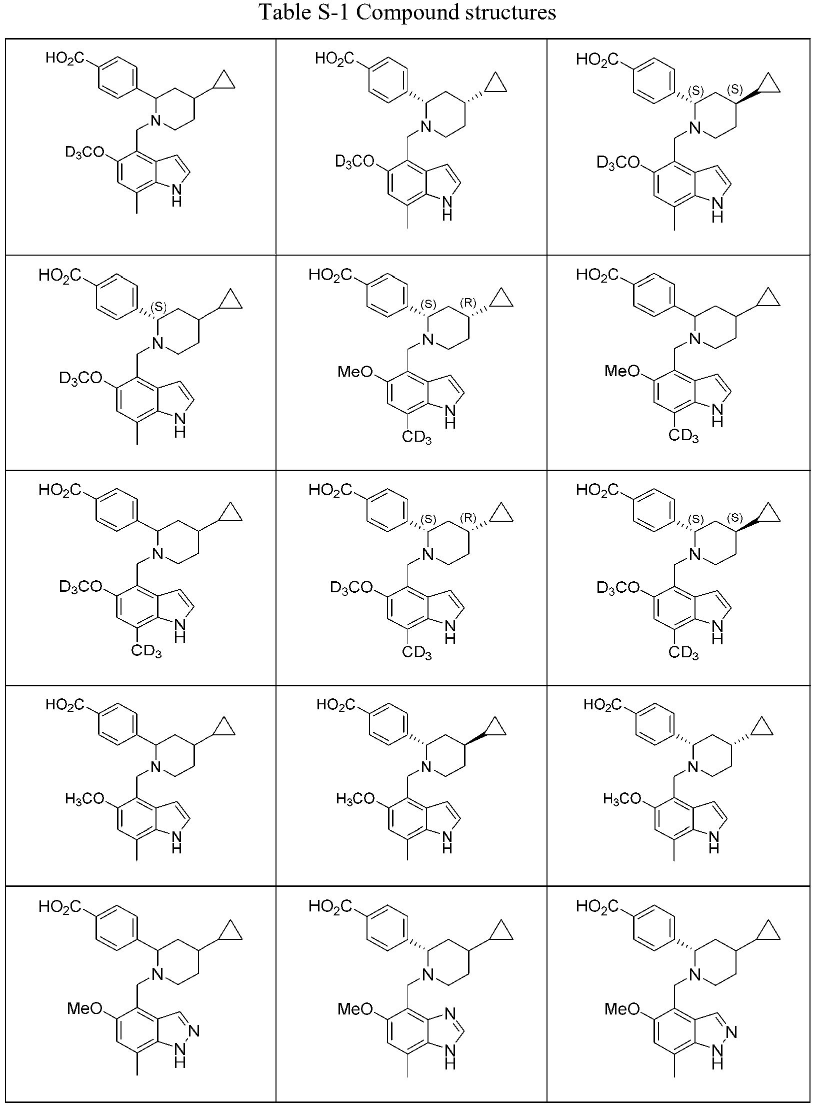

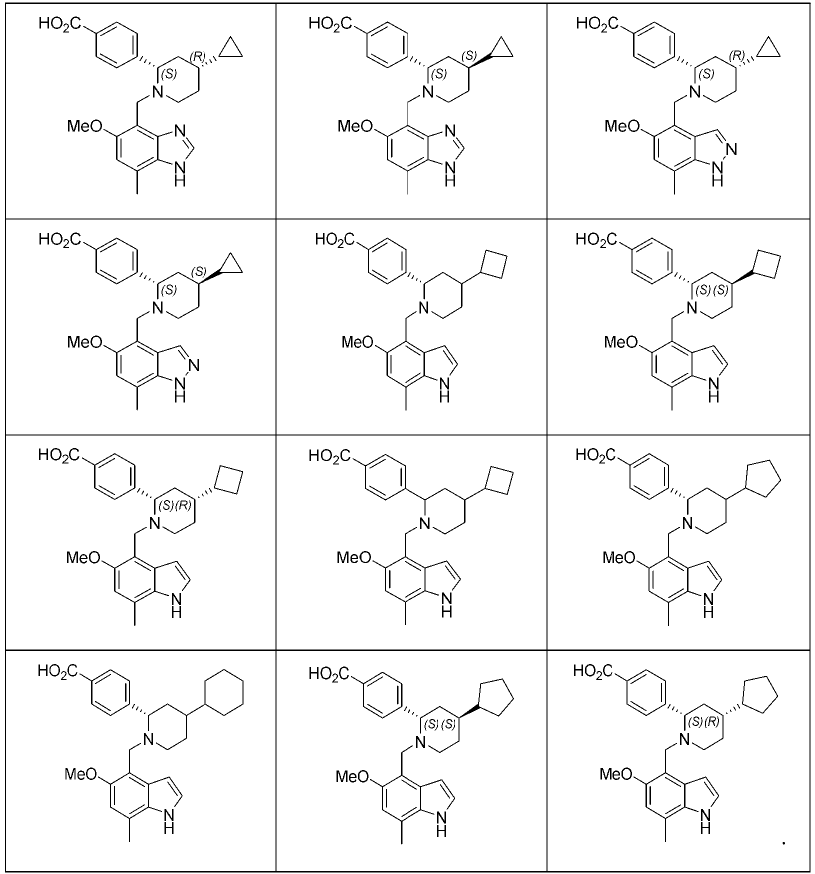

Still further, the compound of formula (I) is selected from the following compounds:

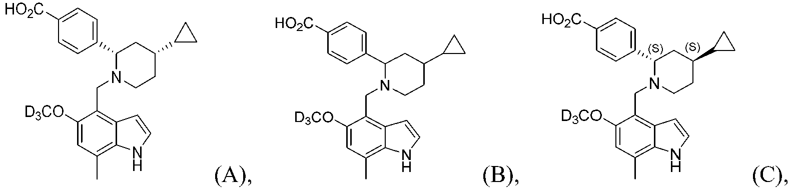

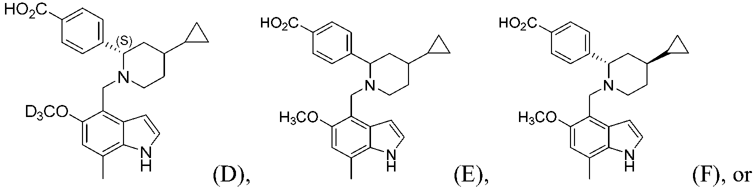

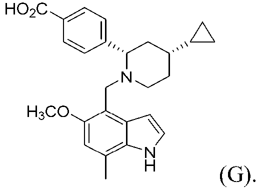

In some embodiments, the use is administering an effective dose of the compound of formula (A), or the stereoisomer, tautomer, pharmaceutically acceptable salt, cocrystal or composition thereof to a mammal.

Further, the pharmaceutically acceptable salt is selected from hydrochloride, succinate, sulfate, maleate, phosphate, tartrate, fumarate, citrate, naphthalene disulfonate, p-toluenesulfonate, methanesulfonate, benzenesulfonate, oxalate, gentisate, succinate, or hydrobromide.

The present invention relates to the complement factor B-mediated disease or condition selected from at least one of: paroxysmal nocturnal hemoglobinuria, primary glomerulonephritis, membranous nephropathy, C3 glomerulonephritis, or atypical hemolytic uremic syndrome.

In some embodiments of the present invention which relate to the use in a drug for preventing or treating a complement factor B-mediated disease and the treatment method, an effective dose of the compound of formula (A) or the stereoisomer, tautomer, pharmaceutically acceptable salt, cocrystal or composition thereof is administered to a mammal, and in terms of the compound of structural formula (A), the amount of the compound is 1-2400 mg/day.

In some embodiments of the present invention which relate to the use in a drug for preventing or treating a complement factor B-mediated disease and the treatment method, in terms of the compound of formula (A), the amount of the compound is 1-2400 mg/day, 1-1000 mg/day, 1-900 mg/day, 1-800 mg/day, 1-700 mg/day, 1-600 mg/day, 1-500 mg/day, 1-400 mg/day, 1-300 mg/day, 1-200 mg/day, 1-100 mg/day, 10-1000 mg/day, 10-900 mg/day, 10-800 mg/day, 10-700 mg/day, 10-600 mg/day, 10-500 mg/day, 10-400 mg/day, 10-300 mg/day, 10-200 mg/day, 10-100 mg/day, 20-500 mg/day, 20-400 mg/day, 20-300 mg/day, 20-250 mg/day, 20-200 mg/day, 20-150 mg/day, 30-100 mg/day, 30-150 mg/day, 30-250 mg/day, 30-300 mg/day, 30-400 mg/day, 40-400 mg/day, 50-100 mg/day, 50-200 mg/day, 50-300 mg/day, 50-400 mg/day, 50-500 mg/day, or 50-600 mg/day.

In some embodiments of the present invention which relate to the use in a drug for preventing or treating a complement factor B-mediated disease and the method for treating a complement factor B-mediated disease, in terms of the compound of formula (A), the compound is administered orally in an amount of 0.5 mg/kg-20 mg/kg.

In some embodiments of the present invention which relate to the use in a drug for preventing or treating a complement factor B-mediated disease and the method for treating a complement factor B-mediated disease, in terms of the compound of formula (A), the compound is administered orally in an amount of 0.5-1 mg/kg, 2-4 mg/kg, 5-10 mg/kg, 11-20 mg/kg, 1-2 mg/kg, 4-8 mg/kg, or 8-16 mg/kg.

In some embodiments of the present invention which relate to the use in a drug for preventing or treating a complement factor B-mediated disease and the method for treating a complement factor B-mediated disease, in terms of the compound of formula (A), the compound is administered orally in an amount of 20 mg-800 mg/day.

In some embodiments of the present invention which relate to the use in a drug for preventing or treating a complement factor B-mediated disease and the method for treating a complement factor B-mediated disease, in terms of the compound of formula (A), the compound is administered orally in an amount of 20 mg-50 mg/day, 60 mg-150 mg/day, 120 mg-300 mg/day, 240 mg-600 mg/day, 320 mg-800 mg/day, 40 mg-100 mg/day, 120 mg-300 mg/day, 240 mg-600 mg/day, 60 mg-150 mg/day, 180 mg-450 mg/day, or 360 mg-800 mg/day.

In some embodiments of the present invention which relate to the use in a drug for preventing or treating a complement factor B-mediated disease and the treatment method, the compound of formula (A), or the stereoisomer, tautomer, pharmaceutically acceptable salt, cocrystal or composition thereof is administered at an interval selected from once a day, twice a day, three times a day, once a week, twice a week, three times a week or once every other day.

In some embodiments of the present invention which relate to the use in a drug for preventing or treating a complement factor B-mediated disease and the method for treating a complement factor B-mediated disease, the compound of formula (A), or the stereoisomer, tautomer, pharmaceutically acceptable salt, cocrystal or composition thereof is administered via a route selected from oral route, injection, drip, transdermal route, sublingual route, parenteral and intraperitoneal route, rectal route, buccal route, nasal spray, nasal drip, inhalation, local delivery, subcutaneous route, intra-adipose route, intra-articular route, intraperitoneal route or intrathecal route, preferably the administration route is oral route, injection and nasal spray.

In some embodiments of the present invention which relate to the use in a drug for preventing or treating a complement factor B-mediated disease and the method for treating a complement factor B-mediated disease, the compound of formula (A), or the stereoisomer, tautomer, pharmaceutically acceptable salt, cocrystal or composition thereof is administered via a route selected from oral route, intravenous injection, intravenous drip, intra-arterial injection, intramuscular injection, subcutaneous injection, intra-articular injection, intraperitoneal injection, intrathecal injection or nasal drip, preferably the administration route is oral route, injection and nasal spray.

Unless stated to the contrary, the terms used in both the description and the claims have the following meanings:
The "pharmaceutical composition" represents a mixture of one or more compounds described herein or a physiologically/pharmaceutically acceptable salt thereof or a stereoisomer, solvate, pharmaceutically acceptable salt or cocrystal thereof and other components comprising physiologically/pharmaceutically acceptable carriers and/or excipients.

The term "stereoisomer" refers to an isomer produced as a result of different spatial arrangement of atoms in molecules, including cis-trans isomers, enantiomers and conformational isomers.

The "effective dose" refers to an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

### Brief Description of the Drawings

Figure 1 shows the effect of Compound A on the hemolysis rate of rabbit red blood cells mediated by serum from PNH patients in test example 3 of the present invention.
Figure 2 shows the effect of Compound A on the urine protein-to-creatinine ratio (UPCR) in rats of the passive Heymann nephritis model in test example 4 of the present invention.
Figure 3 shows the effect of Compound A on the serum AP deposition in rats of the passive Heymann nephritis model in test example 4 of the present invention.
Figure 4 shows the effect of Compound A on the renal pathology in rats of the passive Heymann nephritis model in test example 4 of the present invention.
Figure 5 shows the effect of Compound A on the urine protein-to-creatinine ratio (UPCR) in rats of the IgA nephropathy model in test example 5 of the present invention.
Figure 6 shows the effect of Compound A on the serum C5b-9 deposition in rats of the IgA nephropathy model in test example 5 of the present invention.
Figure 7 shows the effect of Compound A on the serum IgA level in rats of the IgA nephropathy model in test example 5 of the present invention.
Figure 8 shows the effect of Compound A on the renal C3 deposition in rats of the IgA nephropathy model in test example 5 of the present invention.

### Detailed Description of Embodiments

The technical solutions of the present invention will be described in detail below in conjunction with the drawings and examples, but the scope of protection of the present invention includes but is not limited thereto.

### Synthesis example:

The following compounds were prepared according to the methods described in WO 2023020566:
**Compound** A: 4-((2S, 4R)-4-cyclopropyl-1-((5-(methoxy-d3)-7-methyl-1H-indol-4-yl)methyl)piperidin-2-yl)benzoic acid (CAS no.: 2910876-03-6)

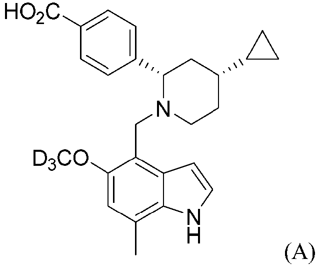

| | | |
|---|---|---|
| Compound B | Compound C | Compound D |
| 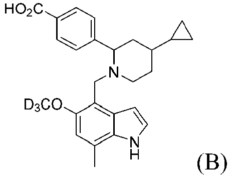 | 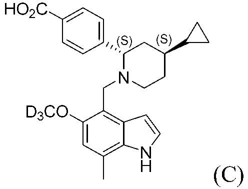 | 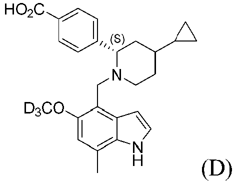 |
| Compound E | Compound F | Compound G |
| 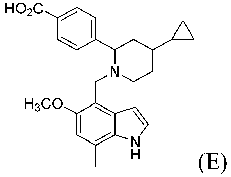 | 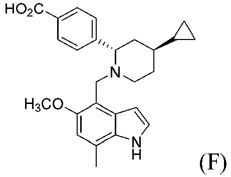 | 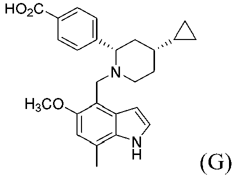 |

### Biological Assay Examples

### Test example 1:

### Test of the effect of Compound A on the enzymatic activity of complement factor B in vitro

1 µM CVF protein (Quidel Corp, Cat#: A600), 1 µM FB protein (Quidel Corp, Cat#: A408) and 300 nM FD protein (Quidel Corp, Cat#: A409) were incubated in an assay buffer (pH = 7.4 PBS, 10 mM MgCl₂, 0.05% (m/v) CHAPS) at room temperature for 3 hours, to obtain a CVFBb complex (C3 convertase). Using Echo 555, 25 nL of a compound was transferred to a reaction plate (Greiner-784075), with the highest concentration of the compound being 600 µM, with 10 concentration points prepared by 3-fold serial dilution , in duplicate wells. The generated CVFBb complex was diluted to 1.67 nM, and 3 µL was added to each well of the reaction plate, followed by incubation with the compound at room temperature for 1 hour. The C3 protein was diluted to 2.5 µM in an assay buffer, and 2 µL was added to each well of the reaction plate to initiate the enzymatic reaction. After incubation at room temperature for 1 hour, a phosphatase inhibitor cocktail was added to terminate the enzymatic reaction. C3a in the samples was quantified using an ELISA kit (HycultBiotech, Cat. #: HK354). IC₅₀ values were calculated by fitting a log(dose)-inhibition curve using GraphPad Prism, to assess the inhibitory effect of the compound on the enzymatic activity of FB.

The results are as shown in Table 1. Compound A can inhibit the enzymatic activity of human complement FB in a dose-dependent manner, with an IC₅₀ of 19.57 nM, indicating that Compound A can significantly inhibit the enzymatic activity of complement B.

**Table 1**

| Compound | IC₅₀ (nM) | IC₉₀ (nM) |
|---|---|---|
| Compound A | 19.57 | 139.9 |

### Test example 2:

### Detection of the inhibitory activity of Compound A in alternative pathway (AP) deposition in human serum

Test example 2: 10 mL of whole blood was collected from healthy volunteers by Shanghai Liquan Hospital into anticoagulant-free blood collection tubes. Blood was allowed to clot in the serum tubes at room temperature (20-25°C) for 30 minutes. The blood sample was centrifuged at 3000 rpm for 10 minutes, and the cell-free serum was transferred to a clean tube. The serum was directly diluted 30-fold with the AP Diluent from the WIESLAB Complement System Alternative Pathway Kit (WIESLAB, Cat#: COMPL AP330 RUO). The compound was diluted with DMSO, with the starting concentration of 5 µM, and 8 concentrations prepared by 4-fold gradient dilution. One 96-well sample plate was prepared, 150 µL of serum was added to an indicated location, then 1.5 µL of the compound was added accordingly, and the mixture was incubated at 37°C for 60 minutes. 100 µL of reaction product was transferred to WIESLAB Complement system Alternative pathway kit assay wells to detect the yield of C5b-9, to evaluate the inhibitory effect of the compound on the activity of the human serum alternative pathway (AP).

The results are shown in Table 2. Compound A can inhibit the complement alternative pathway (AP) deposition in human serum in a dose-dependent manner, with an IC₅₀ of 21.81 nM, indicating that Compound A can significantly inhibit the activation of the alternative pathway in human serum.

**Table 2**

| Name of compound | IC₅₀ (nM) | IC₉₀ (nM) | Max dose (nM) | Inh%@Max dose |
|---|---|---|---|---|
| Compound A | 21.81 | 51.61 | 5000 | 99.6 |

### Test example 3:

### Pharmacodynamic study on the inhibitory effect of Compound A on the rate of hemolysis induced by PNH patient serum

1 mL of 6% rabbit red blood cells (Solarbio, Cat#: S9453) was transferred into a 2 mL EP tube, centrifuged at 9500 g at room temperature for 1 min, and the supernatant was removed. 1 mL of EGTA-Mg²⁺-GVB buffer (CompTech, Cat#: CPT-B103) was added, followed by gentle pipetting for mixing, and viable cells were counted using a cell counter. An appropriate amount of rabbit red blood cells was collected, and the concentration of the rabbit red blood cells was adjusted to 6 × 10⁷ cells/mL using EGTA-Mg²⁺-GVB buffer. The test drug was diluted with normal saline to concentrations of 1000 µM and 60 µM.

The following components were then added to the 96-well plate separately. 1) Test compound wells: 88 µL of EGTA-Mg²⁺-GVB buffer, 2 µL of PNH serum, and 10 µL of test compound dilution. 2) Background wells: 100 µL of EGTA-Mg²⁺-GVB buffer. 3) 100% hemolysis wells: 88 µL of EGTA-Mg²⁺-GVB buffer, 2 µL of PHN serum, and 10 µL of normal saline. After mixing well, the mixture was incubated at 37°C and 200 rpm for 30 min. Each sample was assayed in duplicate. 100 µL of rabbit red blood cells at a concentration of 6 × 10⁷ cells/mL was added to the pre-incubated mixture of test compound and PNH patient serum. The 96-well plate was incubated on a thermostatic incubation microplate shaker at 37°C and 200 rpm for 30 min. The mixture was then centrifuged at 1000 × g at room temperature for 2 min. 100 µL of the supernatant was transferred from each well to a new 96-well plate, and the OD value of the supernatant was measured at 415 nm using a microplate reader (Flexstation 3, Molecular Devices). The hemolysis rate for each sample was calculated using the following equation: Hemolysis rate (%) = 100 × (OD_{mean of test compound wells} - OD_{mean of background wells}) / (OD_{mean of 100% hemolysis wells} - OD_{mean of background wells}).

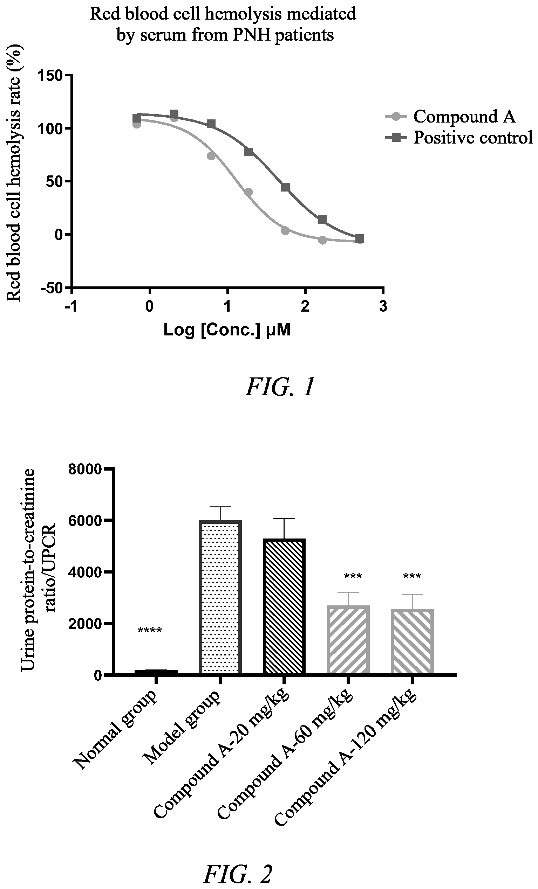

The results are as shown in Figure 1. Compound A can inhibit the red blood cell hemolysis mediated by PNH patient serum in a dose-dependent manner, with an IC₅₀ of 12.71 µM. The positive control (LNP023) exhibited an IC₅₀ of 44.84 µM in inhibiting PNH-mediated red blood cell hemolysis. The inhibitory effect of Compound A on complement-mediated red blood cell hemolysis was superior to that of the positive control (LNP023).

### Test example 4:

### Pharmacodynamic effect of Compound A on anti-serum Fx1A-induced passive Heymann nephritis in rats

This experiment investigated the protective effect of Compound A in a rat model of Fx1A-induced Heymann nephritis.

On Day -1, 60 male SD rats were evenly divided into 6 groups based on the body weight, with 10 rats per group. On Day 0, except for the normal group (blank serum being administered to the normal group), all other rats were slowly injected with anti-rat Fx1A serum via the tail vein at a dose of 0.8 mL/100 g. Corresponding compound or vehicle was administered 4 hours after injection. The rats were observed for any signs of discomfort or reaction to the injection within 4 hours. On Day 0-Day 14: the corresponding compound or vehicle were administered to all animals based on the body weight, at a dosing volume of 5 mL/kg, twice daily. On Day 13, the animals were placed into metabolic cages after dosing. On Day 14, 24-hour urine samples were collected, and the volume was recorded for the measurement of total protein (TP) and urine creatinine. Blood samples were collected from the jugular vein of rats prior to dosing. 1 hour after dosing, the rats were euthanized via CO₂, and blood samples were collected from the heart for the determination of serum creatinine, urea, and AP activity. Kidneys were perfused with pre-chilled 0.9% normal saline until complete perfusion was achieved. Bilateral kidneys were harvested and weights were recorded. The middle portion of the left kidney was fixed in formalin for pathological examination.

The results are as shown in Figure 2. Compound A at doses of 20, 60, and 120 mg/kg reduced UPCR, demonstrating favorable protective effects on the kidney.

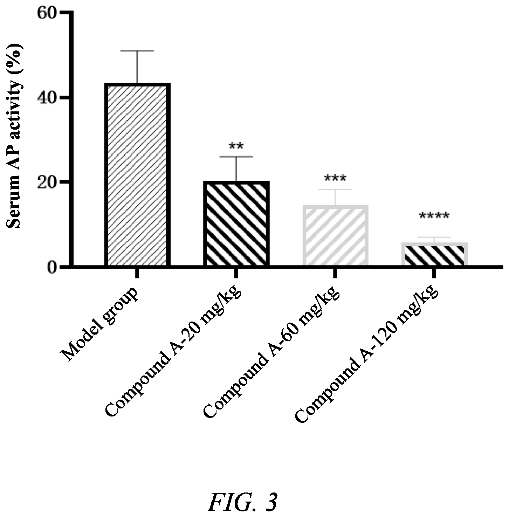

As shown in Figure 3, Compound A at 20, 60, and 120 mg/kg inhibited the activity of the serum complement marker AP in rats in a dose-dependent manner, indicating that Compound A exerts its protective effects on the kidney by suppressing AP activity.

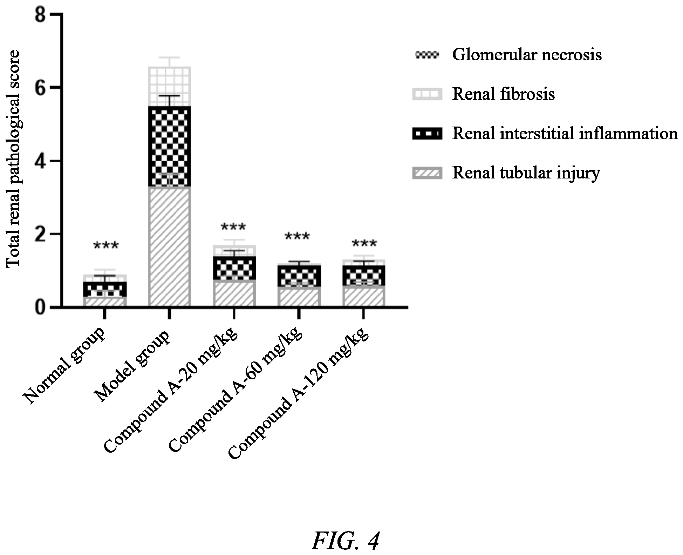

As shown in Figure 4, the effects of Compound A on renal pathology of rats were detected in a rat model of Heymann nephritis. The results demonstrated that Compound A reduced renal tubular injury, renal fibrosis, renal interstitial inflammation, and total renal pathological scores at 20, 60, and 120 mg/kg in a dose-dependent manner.

### Test example 5:

### This experiment investigated the protective effect of Compound A in a rat model of BSA-induced IgA nephritis.

Before the experiment, 95 Wistar rats were pre-screened using urine protein test strips. Animals with abnormal values were excluded, and 55 rats were retained for the experiment. 5 animals with normal urine protein levels were randomly selected as the G1 normal group. Subsequently, modeling was initiated in the remaining animals with normal urine protein levels. All modeled rats received intravenous tail injection of BSA solution starting on Day 0 at an injection dose of 0.5 mg per rat, 1 mL per administration, three times weekly for 5-7 consecutive weeks. Following tail vein injection of BSA, the modeled animals were grouped according to changes in total urine protein content measured by a biochemical analyzer. The modeled rats were divided into 5 groups with 10 rats per group, plus 5 rats in the normal group, i.e. 55 rats in total. Dosing was initiated after grouping. On Day 49, 24-hour urine samples were collected using rat metabolic cages, and the total urine volume was recorded. The concentrations of total protein and creatinine in urine were determined by a biochemical analyzer, and the total 24h urine protein level and UPCR were calculated based on the urine volume. At the experimental endpoint on Day 54, all experimental animals were anesthetized, and blood samples were collected via the abdominal aorta. Contents of C5b-9 and IgA were measured using ELISA kits. After blood collection, the animals were perfused with normal saline, and the right kidney was harvested for renal pathological examination, including determination of renal C3 deposition.

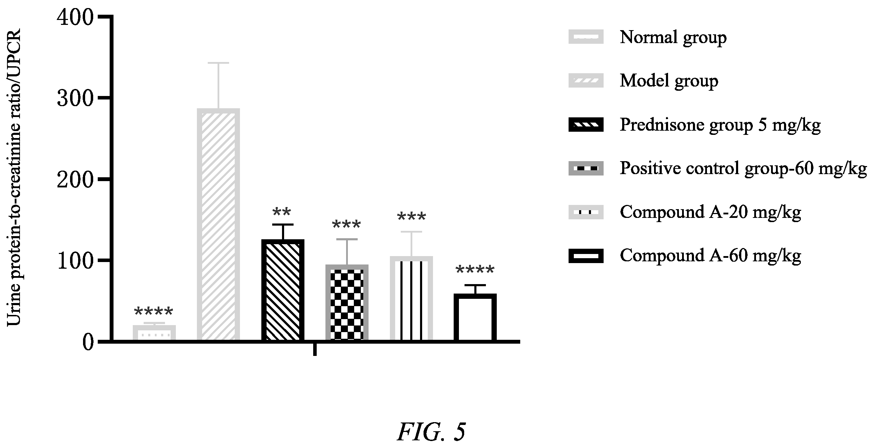

The results are as shown in Figure 5. On Day 49, compared with the model group, Compound A at 20 and 60 mg/kg reduced UPCR in rats in a dose-dependent manner, exhibiting favorable protective effects on the kidney that were superior to those of the positive control (LNP023) group at the same doses.

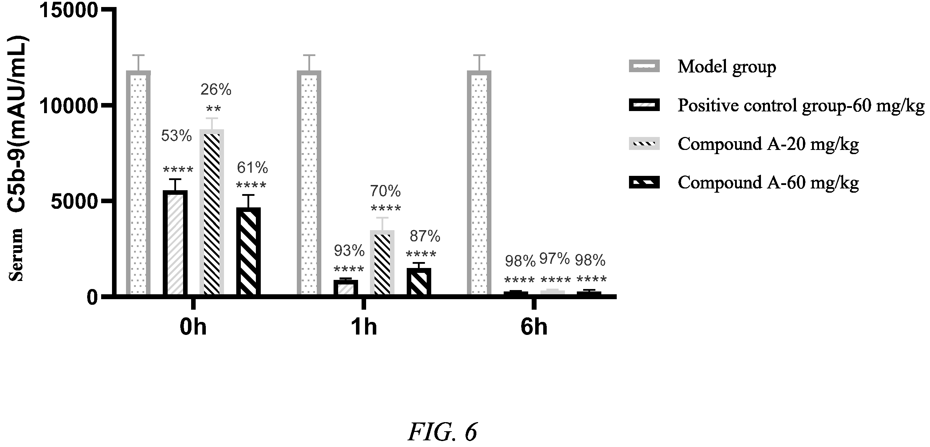

As shown in Figure 6, the levels of the serum complement marker C5b-9 were determined at the experimental endpoint. The results demonstrated that Compound A at 20 and 60 mg/kg inhibited serum C5b-9 deposition in rats in a dose-dependent manner, with the inhibitory effect enhanced as the duration of administration increased. These findings indicate that Compound A exerts protective effects on the kidney by inhibiting the activity of the entire complement pathway.

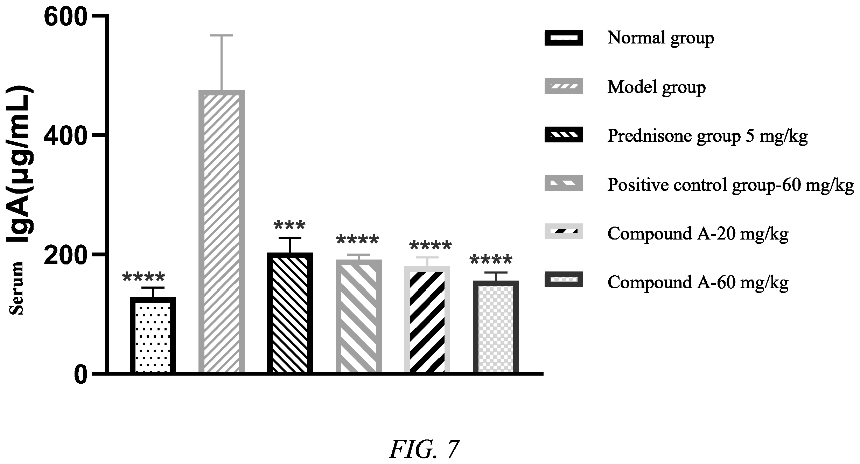

As shown in Figure 7, Compound A at 20 and 60 mg/kg significantly reduced the content of IgA as the serum marker of the IgA nephritis model, and the effects were superior to those of the positive control (LNP023) group at the same doses.

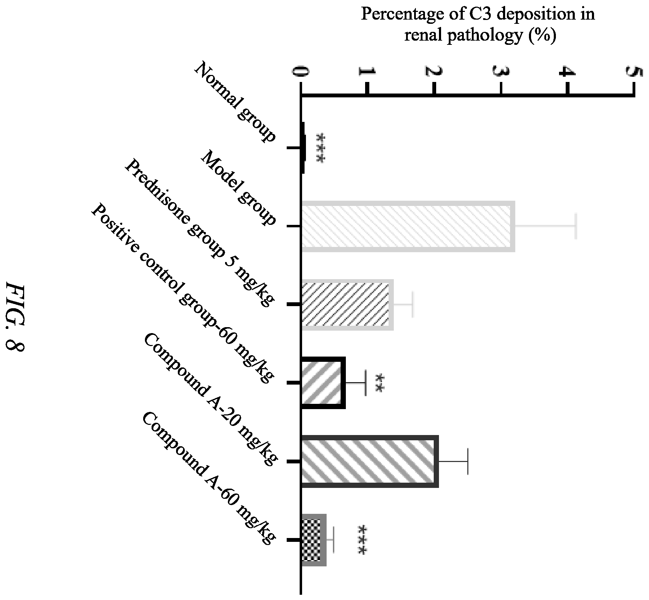

As shown in Figure 8, Compound A at 60 mg/kg inhibited C3 deposition in rat kidneys in a dose-dependent manner, and the effect was superior to that of the positive control (LNP023) group at the same dose.

### Clinical studies:

### Clinical study 1: a multi-center, randomized, open-label phase II clinical study to evaluate the efficacy and safety of Compound A tablets in patients with paroxysmal nocturnal hemoglobinuria

### 1. Overall study design

This study is a multi-center, randomized, open-label phase II trial that plans to enroll approximately 45 patients with paroxysmal nocturnal hemoglobinuria who have not received complement inhibitor therapy. Subjects who met the enrollment conditions will be randomly assigned in a 1 : 1 : 1 ratio to three treatment groups receiving Compound A tablets: dose group A, dose group B, and dose group C. An interim data analysis will be performed after all subjects have completed 12 weeks of study treatment. During the interim analysis period, enrolled subjects will continue to receive their assigned dose of study treatment up to 24 weeks. At the end of the treatment observation period, subjects deemed by the investigator to have no therapeutic benefit will enter a dose-tapering period and a safety follow-up period. All other subjects will enter an independent extended dosing study after providing informed consent.

The trial duration consists of a screening period (up to 8 weeks), a treatment observation period (24 weeks), a dose-tapering period (1 week), and a safety follow-up period (3 weeks).

### Screening period:

Following signature of the informed consent form, subjects will complete visits, examinations, and vaccinations from Day -56 to Day -2 at the screening period. Subjects who have not previously been vaccinated against meningococcus and *Streptococcus pneumoniae* should complete vaccination at least 2 weeks prior to the first dose. Relevant laboratory tests and assessments will be performed again for subjects on the day prior to the first dose (Day -1).

### Treatment observation period:

Subjects who met all inclusion criteria and none of the exclusion criteria, as assessed by the investigator, will be randomized on D1. If the first dose of Compound A tablets was initiated fewer than 2 weeks after vaccination, subjects should receive antibiotic prophylaxis until at least 2 weeks post vaccination.

During the treatment observation period, subjects in group A received treatment of Compound A tablets at 75 mg BID for 4 weeks starting on D1, followed by 125 mg BID for 20 weeks of treatment. If the reduction in LDH level was < 40% on D15 relative to baseline, the dose will be escalated to 125 mg BID for treatment. Subjects in group B received a fixed dose of treatment of Compound A tablets at 100 mg BID starting on D1. Subjects in group C received a fixed dose of treatment of Compound A tablets at 200 mg QD starting on D1. The total duration of the treatment observation period was 24 weeks. During treatment, dose adjustments may be made (e.g., dose reduction from 125 mg BID to 100 mg BID, or from 100 mg BID to 75 mg BID) for safety or tolerability reasons, following consultation between the investigator and the sponsor.

### Extended dosing study:

After completing the 24-week treatment observation period, subjects with no safety or tolerability issues will enter a separate extended dosing study upon providing informed consent. Subjects participating in the extended dosing study should continue to receive study medication in accordance with the provisions of the respective study protocol.

### Dose tapering period:

After completing the 24-week treatment observation period, subjects deemed by the investigator to have no therapeutic benefit will enter a 1-week dose tapering period. The dose will be reduced to 50 mg QD for 4 days, then further reduced to 25 mg QD for 3 days before discontinuation.

### Safety follow-up period:

After completing the dose-tapering period, subjects will enter a 3-week safety follow-up period. Subjects will be discontinued from the study after completing the protocol-specified safety indicator collection and assessments at the final visit.

### 2. Study population

### 2.1 Inclusion criteria

### Subjects who meet all of the following criteria can be enrolled in this study:

(1) age ≥ 18 years at screening, male or female;
(2) confirmed diagnosis of PNH by flow cytometry (the proportion of granulocyte clone being > 10%) within 6 months prior to screening; flow cytometry results detected at V1 during the screening period being acceptable;
(3) no prior treatment with complement inhibitors;
(4) serum LDH > 1.5 × upper limit of normal (ULN) as measured at V1 and V2 during the screening period;
(5) hemoglobin levels assessed by laboratory testing meeting one of the following criteria: (1) hemoglobin (local laboratory) < 100 g/L at V1 and V2 during the screening period; (2) if the subject receives red blood cell transfusion treatment for PNH-related anemia during the screening period, hemoglobin (local laboratory) at V1 must be < 100 g/L;
(6) vaccination against *Neisseria meningitidis* and *Streptococcus pneumoniae* at least 2 weeks before the first dose, and complying with the protocol-specified vaccination requirements; if study drug treatment must start fewer than 2 weeks after vaccination, prophylactic antibiotic therapy must be initiated and continued until at least 2 weeks post vaccination;
(7) female subjects of childbearing potential who must have a negative pregnancy test during the screening period, and must agree not to attempt conception, not to donate ova, and to use an effective contraceptive measure from the time of signing the informed consent form until at least 30 days after the last dose of study drug. male subjects of childbearing potential who must agree not to donate sperm and to use an effective contraceptive measure with their female partners from the time of signing the informed consent form until at least 90 days after the last dose of study drug; and
(8) understanding the study procedures and methods, volunteering to participate in this trial, and signing the informed consent form.

### 2.2 Exclusion criteria

### Subjects who met any of the following criteria were ineligible for enrollment in this study:

(1) participation in another interventional clinical trial within 3 months prior to screening, or ongoing participation in the follow-up period of a clinical study or within 5 half-lives of an investigational drug at screening (whichever is longer);
(2) known or suspected hereditary or acquired complement deficiency;
(3) a history of currently active primary or secondary immunodeficiency;
(4) a history of splenectomy;
(5) a history of bone marrow/hematopoietic stem cell transplantation or solid organ (e.g., heart, lung, kidney, liver) transplantation;
(6) a history of malignancy within 5 years prior to screening (excluding cured basal cell carcinoma of the skin or cervical carcinoma in situ);
(7) a previous history of recurrent invasive infection caused by encapsulated bacteria (e.g., *Neisseria meningitidis*, *Streptococcus pneumoniae*, etc.) or *Mycobacterium tuberculosis*;
(8) subjects who have a history of having or suspected systemic active bacterial, viral or fungal infection within 2 weeks prior to the first dose of study drug, and are judged by the investigator to be unsuitable for participation in the study;
(9) subjects having laboratory evidence of bone marrow failure at V1 or V2 during the screening period (local laboratory): reticulocytes < 100 × 109/L, or platelets < 30 × 109/L, or neutrophils < 0.5 × 109/L;
(10) subjects having positive test results for hepatitis B surface antigen (HBsAg), hepatitis C virus antibody (HCV-Ab), human immunodeficiency virus antibody (HIV-Ab), or *Treponema pallidum* antibody (TP-Ab) during the screening period;
(11) subjects receiving treatment of any of the following drugs before screening, with the following duration of treatment at stable doses of the drugs:
   (a) erythropoietin for less than 8 weeks;
   (b) immunosuppressants (including but not limited to cyclosporine, tacrolimus, mycophenolate or mycophenolic acid, cyclophosphamide, methotrexate, etc.) for less than 8 weeks;
   (c) hypoxia-inducible factor prolyl hydroxylase inhibitors (HIF-PHIs) for less than 8 weeks;
   (d) systemic glucocorticoids for less than 4 weeks;
   (e) vitamin K antagonists (e.g., warfarin) with a stable international normalized ratio for less than 4 weeks;
   (f) low molecular weight heparin or oral anticoagulants (e.g., rivaroxaban) for less than 4 weeks;
   (g) iron supplements, vitamin B12, and folic acid for less than 4 weeks;
   (h) androgens for less than 4 weeks;
(12) presence of severe comorbidities, including but not limited to severe hepatic impairment, severe renal disease (e.g., eGFR < 30 mL/min/1.73 m2), unstable angina pectoris, or a history of other hematological disease (e.g., chronic anemia unrelated to PNH), as judged by the investigator to render the subject ineligible for participation in the study;
(13) suspected hypersensitivity to any component of the study drug or to drugs of the same class;
(14) pregnant or lactating female subjects; and
(15) any other conditions that may interfere with study conduct, impose additional risks to the subject, or render the subject ineligible for participation in the study as assessed by the investigator.

### 2.3 Study completion

A subject will be considered to have completed the study after signing the informed consent form and finishing the protocol-specified final visit or trial flow. The entire study will be considered completed after the last subject has finished the protocol-specified final visit or trial flow.

### 3. Study medication

### 3.1 Investigational medicinal products

Compound A tablets were manufactured and supplied by Sichuan Haisco Pharmaceutical Co., Ltd., and prepared according to the method recorded in WO 2024175028 A1.

Specification: 25 mg and 100 mg (both calculated as based on C₂₆H₂₇D₃N₂O₃).

Administration method: oral administration. The drugs may be taken on an empty stomach or after meals. The dosing frequency was BID (once in the morning and once in the evening daily) or QD (once in the morning daily).

Storage conditions: tightly closed storage at room temperature.

### 3.2 Administration method of study drugs

### 3.2.1 Planned dosing method

All eligible subjects will receive the investigational medicinal product on the day of the first dose (D1). The investigator shall inform subjects of the administration requirements and precautions during study treatment, including but not limited to: swallowing drugs whole without chewing, adhering to the protocol-specified daily dosing frequency and number of tablets, and maintaining consistent dosing times as much as possible.

The dosing regimens and administration methods for the different treatment groups during the study treatment observation period were as follows:
Group A: Subjects received treatment of Compound A tablets at 75 mg BID for 4 weeks starting on D1, followed by 125 mg BID for 20 weeks of treatment. If the reduction in LDH level was < 40% on D15 relative to baseline, the dose will be escalated to 125 mg BID for treatment.
Group B: Subjects received a fixed dose of treatment of Compound A tablets at 100 mg BID starting on D1, for 24 weeks.
Group C: Subjects received a fixed dose of treatment of Compound A tablets at 200 mg QD starting on D1, for 24 weeks.

The total duration of the treatment observation period was 24 weeks. During treatment, dose adjustments may be made (e.g., dose reduction from 125 mg BID to 100 mg BID, or from 100 mg BID to 75 mg BID) for safety or tolerability reasons, following consultation between the investigator and the sponsor.

Dose tapering period:
The dose was reduced to 50 mg QD for 4 days, then further reduced to 25 mg QD for 3 days before discontinuation.

### 3.2.2 Extended treatment

After completing the 24-week treatment observation period, subjects with no safety or tolerability issues and deemed by the investigator to have therapeutic benefits will enter a separate extended dosing study upon providing informed consent. Subjects participating in the extended dosing study should continue to receive study medication in accordance with the provisions of the respective study protocol.

### 4. Clinical trial flow

Subjects should visit the study center at the visit time notified in advance by the investigator. If any of the following requirements was not met, the visit shall be rescheduled:
- Subjects should be in a fasting state (at least 8 hours of fasting) for all routinely scheduled clinical visits.
- Subjects should avoid a high-fat, high-calorie diet on the day prior to study visits.
- Subjects should avoid alcohol, tobacco, and caffeine within 8 hours prior to study visits.

In this study, blood samples for hemoglobin and reticulocytes (including V2, the treatment observation period, and early termination visits), free hemoglobin, C3 fragment deposition, and PNH clone size shall be sent to a central laboratory for detection to evaluate efficacy. Considering the stability time window for sample detection at the central laboratory, blood routine test samples and reticulocyte samples at all visits shall also be detected in the local laboratory in parallel.

### 5. Evaluation indicators

### 5.1 Evaluation of efficacy

### 5.1.1 Primary endpoint

After 24 weeks of treatment, the proportion of subjects who achieved an increase in hemoglobin (Hb) concentration of ≥ 20 g/L relative to baseline among those who did not receive red blood cell transfusions after 4 weeks of dosing.

### 5.1.2 Secondary endpoints

- After 24 weeks of treatment, the proportion of subjects who achieved the Hb level of ≥ 120 g/L among subjects who did not receive red blood cell transfusions after 4 weeks of dosing;
- after 24 weeks of treatment, the proportion of subjects with ≥ 60% reduction in lactate dehydrogenase (LDH) relative to baseline or LDH below the upper limit of normal;
- after 24 weeks of treatment, changes relative to baseline in Hb and reticulocyte count in subjects who did not receive red blood cell transfusions after 4 weeks of dosing;
- after 24 weeks of treatment, changes relative to baseline in LDH, indirect bilirubin, and free hemoglobin;
- the proportion of subjects who did not receive red blood cell transfusions during the treatment period from week 4 to week 24;
- changes relative to pre-dosing in the mean number of red blood cell units transfused per week in subjects during the treatment period from week 4 to week 24;
- after 24 weeks of treatment, changes relative to baseline in the PNH red blood cell clone proportion in subjects who did not receive red blood cell transfusions after 4 weeks of dosing;
- after 24 weeks of treatment, changes relative to baseline in C3 complement fragment deposition on PNH red blood cells; and
- after 24 weeks of treatment, changes relative to baseline in the Functional Assessment of Chronic Illness Therapy-Fatigue (FACIT-F) scale score.

### 5.2 Evaluation of safety

### Safety indicators include:

- incidence and severity of adverse events;
- incidence of thromboembolic events; and
- laboratory tests, electrocardiogram, vital signs, physical examination and the like.

### 5.3 Evaluation of pharmacokinetics/pharmacodynamics

### Pharmacokinetic indicators include:

- concentration of Compound A in plasma; and
- changes relative to baseline in alternative complement pathway (AP) activity, Bb concentration, and plasma sC5b-9 concentration.

### 6. Adverse events

**Adverse events (AE):** refer to all adverse medical events occurring in subjects in this clinical study. AEs can be manifested as symptoms, signs, disease, or abnormal laboratory test results, but may not necessarily have a causal relationship with the investigational medicinal product. Therefore, AEs may be any unfavorable, unintended symptom, sign, test value, or disease (either newly occurring condition or exacerbation of a pre-existing condition).

All adverse events that occur after the subject uses the investigational medicinal product are defined as TEAEs.

### AEs do not include:

- non-exacerbated chronic disease already present prior to participation in the clinical study;
- scheduled medical examinations or surgical procedures prior to enrollment in the clinical study; and
- excessive use of the investigational medicinal product or concomitant medications that did not result in any sign or symptom.

### 7. Indicator analysis

### 7.1 Primary efficacy indicator analysis

The primary endpoint of the study was, after 24 weeks of treatment, the proportion of subjects who achieved an increase in hemoglobin (Hb) concentration of ≥ 20 g/L relative to baseline among those who did not receive red blood cell transfusions after 4 weeks of dosing. The study employed a historical control design, and the analysis was conducted to assess whether the primary endpoint was significantly higher than the target value (15%) of the historical control.

For the primary efficacy endpoint, the following statistical hypotheses were established:
Null hypothesis: ${π}_{T} - {π}_{0} \leq 0$
Alternative hypothesis: ${π}_{T} - {π}_{0} > 0$
where *π_{T}* refers to, after 24 weeks of treatment, the proportion of subjects who achieved an increase in hemoglobin (Hb) concentration of ≥ 20 g/L relative to baseline among those who did not receive red blood cell transfusions after 4 weeks of dosing; and πₒ is 0.15. After 24 weeks of treatment, the proportion of subjects who achieved an increase in hemoglobin (Hb) concentration of ≥ 20 g/L relative to baseline among those who did not receive red blood cell transfusions after 4 weeks of dosing; its 95% confidence interval; and the corresponding P-value derived from the binomial distribution test with a threshold of 15% were calculated.

The primary analysis set was based on the FAS, and meanwhile the sensitivity analysis was performed using PPS.

For other sensitivity analysis methods, reference can be made to SAP for details.

### 7.2 Secondary efficacy indicator analysis

Statistical analysis was generally performed based on the FAS.
- After 24 weeks of treatment, the proportion of subjects who achieved an Hb level of ≥ 120 g/L among those who did not receive red blood cell transfusions after 4 weeks of dosing.

After 24 weeks of treatment, the proportion of subjects who achieved an Hb level of ≥ 120 g/L among those who did not receive red blood cell transfusions after 4 weeks of dosing, and the corresponding 95% confidence interval thereof were calculated, using methods similar to those for the primary efficacy indicator.
- After 24 weeks of treatment, the proportion of subjects with ≥ 60% reduction in LDH relative to baseline or LDH below the upper limit of normal.

After 24 weeks of treatment, the proportion of subjects with ≥ 60% reduction in LDH relative to baseline or LDH below the upper limit of normal, and the corresponding 95% confidence interval thereof were calculated by group using methods similar to those for the primary efficacy indicator.
- After 24 weeks of treatment, changes relative to baseline in Hb and reticulocyte count in subjects who did not receive red blood cell transfusions after 4 weeks of dosing.

Changes relative to baseline in Hb and reticulocyte count at each post-baseline analysis visit were analyzed using a mixed-effects model for repeated measures (MMRM). The model included analysis visit and treatment group as fixed effects, subjects as a random effect, and baseline values of Hb and reticulocyte count as covariates. Least squares means (LSMean), standard errors (SE), and corresponding 95% CI were calculated. The model was constructed using an unstructured (UN) covariance structure. If the model failed to converge, Toeplitz (TOEP), first-order autoregressive (AR(1)), or compound symmetry (CS) covariance structures would be considered sequentially until convergence was achieved. Line graphs were plotted with weeks as the horizontal axis to show the baseline and least squares means of changes in Hb and reticulocyte count at each post-baseline analysis visit relative to baseline.
- After 24 weeks of treatment, changes relative to baseline in LDH, indirect bilirubin, and free hemoglobin.

Similar to the analysis for the changes relative to baseline in Hb and reticulocyte count, using the mixed-effects model for repeated measures (MMRM), changes relative to baseline in LDH, indirect bilirubin, and free hemoglobin were analyzed by treatment group separately, and the corresponding least squares means and the 95% confidence intervals thereof were calculated.
- The proportion of subjects who did not receive red blood cell transfusions during the treatment period from week 4 to week 24.

The proportion of subjects who did not receive red blood cell transfusions during the treatment period from week 4 to week 24 and the 95% confidence interval thereof were calculated using methods similar to those for the primary efficacy indicator.
- Changes relative to pre-dosing in the mean number of red blood cell units transfused per week in subjects during the treatment period from week 4 to week 24.

Similar to the analysis for the changes relative to baseline in Hb and reticulocyte count, using the mixed-effects model for repeated measures (MMRM), changes relative to pre-dosing in the mean number of red blood cell units transfused per week during the treatment period from week 4 to week 24 were analyzed by group separately, and the corresponding least squares means and the 95% confidence intervals thereof were calculated.
- After 24 weeks of treatment, changes relative to baseline in the PNH red blood cell clone proportion in subjects who did not receive red blood cell transfusions after 4 weeks of dosing.

Similar to the analysis for the changes relative to baseline in Hb and reticulocyte count, using the mixed-effects model for repeated measures (MMRM), changes relative to baseline in the PNH red blood cell clone proportion in subjects who did not receive red blood cell transfusions after 4 weeks of dosing were analyzed by group separately, and the corresponding least squares means and the 95% confidence intervals thereof were calculated.
- After 24 weeks of treatment, changes relative to baseline in C3 complement fragment deposition on PNH red blood cells.

Similar to the analysis for the changes relative to baseline in Hb and reticulocyte count, using the mixed-effects model for repeated measures (MMRM), changes relative to baseline in C3 complement fragment deposition on PNH red blood cells were analyzed, and the corresponding least squares means and the 95% confidence intervals thereof were calculated.
- After 24 weeks of treatment, changes relative to baseline in the Functional Assessment of Chronic Illness Therapy-Fatigue (FACIT-F) scale score.

Similar to the analysis for the changes relative to baseline in Hb and reticulocyte count, using the mixed-effects model for repeated measures (MMRM), changes relative to baseline in the Functional Assessment of Chronic Illness Therapy-Fatigue (FACIT-F) scale score were analyzed, and the corresponding least squares means and the 95% confidence intervals thereof were calculated.

### 7.3 Safety analysis

Unless otherwise specified, descriptive statistical analysis was performed on safety data in SS.

Adverse events were coded using MedDRA Version 27.0 or later, and summarized by Preferred Term (PT) and System Organ Class (SOC). The number of subjects, number of events, and percentages of subjects with adverse events were reported. Adverse events were summarized and tabulated by their relationship to the investigational medicinal product and severity. Adverse events related to the investigational medicinal product, adverse events leading to temporary interruption of medication, adverse events leading to dose reduction, adverse events leading to permanent discontinuation of medication, adverse events leading to trial withdrawal, serious adverse events, and deaths were separately summarized and tabulated.

Results of laboratory tests (blood routine test, urine routine test, blood biochemistry, coagulation function, etc.), electrocardiography, and vital signs at scheduled assessment time points were summarized by group. Cross-tabulations were used to describe changes in the clinical judgment of post-dose visit results relative to baseline for each indicator. All test results and abnormal test results were tabulated by subject.

Physical examination data were tabulated by subject, with abnormal results summarized.

Using descriptive statistics, the incidence of thromboembolic events were described by group separately, and data on thrombus occurrence were tabulated by subject.

### 7.4 Pharmacokinetic/pharmacodynamic analysis

### • Pharmacokinetic analysis

**Pharmacokinetic concentration analysis:** PK concentration data were statistically summarized and tabulated by dose group, according to each scheduled sampling time point defined in the protocol. Mean drug concentration-time profiles (linear and semi-logarithmic plots) were plotted for each dose group at V5 and V8. Individual PK concentration data of subjects at V5 and V8 were plotted as drug concentration-time profiles (linear and semi-logarithmic plots) by dose group according to actual sampling times.

**Pharmacokinetic parameter analysis:** Pharmacokinetic parameters were calculated using a non-compartmental model. The Power model was used to evaluate the linear relationship between main PK parameters (AUC₀₋ₜₐᵤ, Cₘₐₓ) and the dose.

### • Pharmacodynamic analysis

Measured values of AP, Bb, and sC5b-9, as well as their changes relative to baseline, were statistically summarized and tabulated by dose group, according to each scheduled sampling time point defined in the protocol. Meanwhile, individual plots and mean plots of the percent change relative to baseline over time were plotted by dose group.

### 8. Conclusions of the study

The study results demonstrate that Compound A tablets exhibit favorable efficacy and safety in the treatment of paroxysmal nocturnal hemoglobinuria.

### Clinical study 2: a randomized, double-blind, placebo-controlled, parallel-group phase II clinical trial to evaluate the efficacy and safety of Compound A tablets in the treatment of primary IgA nephropathy

### 1. Study objectives and endpoints

**Table 6. Study objectives and study endpoints**

| Objectives | Endpoints |
|---|---|
| Primary study objective | Primary endpoint |
| To evaluate the efficacy of Compound A tablets for treating patients with primary IgA nephropathy | Ratio of 24h-UPCR after 12 weeks of treatment relative to baseline |
| Secondary study objectives | Secondary endpoints |
| To evaluate the effect of Compound A tablets on renal function indicators in patients with primary IgA nephropathy | Ratio of 24h-UPCR after 24 weeks of treatment relative to baseline; |
| | Ratio of 24h-UPCR relative to baseline at each clinical visit during the treatment period |
| | Ratio of 24h-UPE relative to baseline at each clinical visit during the treatment period |
| | Changes relative to baseline in estimated glomerular filtration rate at each clinical visit during the treatment period |
| | Changes relative to baseline in serum creatinine at each clinical visit during the treatment period |
| | Ratios of FMV UPCR and UACR relative to baseline at each clinical visit during the treatment period |
| | Changes relative to baseline in the Functional Assessment of Chronic Illness Therapy-Fatigue (FACIT-F) scale score at each clinical visit during the treatment period; |
| To evaluate the safety of Compound A tablets in patients with primary IgA nephropathy | Adverse events, vital signs, physical examination, 12-lead electrocardiogram, laboratory tests, etc., during the study period |
| To evaluate the pharmacokinetic and pharmacodynamic profiles of Compound A tablets in patients with primary IgA nephropathy | Pharmacokinetic profiles of Compound A in plasma; and changes relative to baseline in complement alternative pathway (AP) activity, plasma Bb level, and plasma and urine sC5b-9 level at each clinical visit. |

### 2. Overall design

This study was a multicenter, randomized, double-blind, placebo-controlled, parallel-group phase II clinical trial designed to enroll a total of 80 patients with primary IgA nephropathy. The study included a screening period (up to 2 weeks), a run-in period (at least 4 weeks), a 12-week core treatment period, a 12-week extended treatment period, and a 4-week safety follow-up period.
- Screening period:
   Screening began after subjects provided informed consent forms. The screening period was a maximum of 2 weeks. Subjects who completed the examination during the screening period and met the criteria for entry into the run-in period, as assessed by the investigator, can proceed to the run-in period.
- Run-in period:
   Following entry into the run-in period, subjects must complete vaccination against *Neisseria meningitidis* and *Streptococcus pneumoniae* at least 2 weeks prior to the first dose of medication.

For subjects naïve to optimized RAS blocker therapy: the optimized RAS blocker therapy was initiated immediately after the subject entered the run-in period. The treatment regimen was determined by the investigator based on clinical practice (see appendix 3 for reference). After reaching the maximum recommended dose or maximum tolerated dose, the dose must remain stable for at least 4 weeks. The duration of the optimized RAS blocker therapy shall be no less than 12 weeks. Subjects who met the randomization criteria at the end of the run-in period can undergo randomization.

For subjects for whom the optimized RAS blocker therapy had been previously initiated but had not reached the maximum recommended dose or maximum tolerated dose: for subjects for whom the RAS blocker had not reached the maximum recommended dose or maximum tolerated dose (with a traceable treatment regimen) prior to entering the run-in period, the investigator continued to adjust the RAS blocker dose to the maximum recommended dose or maximum tolerated dose based on clinical practice, followed by maintaining the current dose stable for 4 weeks. Those subjects who met the randomization criteria at the end of the run-in period can undergo randomization.

For subjects who had been previously treated by the optimized RAS blocker therapy and for whom the maximum recommended dose or maximum tolerated dose had been reached: the current dose was maintained stable for 4 weeks after the subjects entered the run-in period. Those subjects who met the randomization criteria at the end of the run-in period can undergo randomization.
- Core treatment period:
   Subjects who met the randomization criteria were randomly assigned in a 1 : 1 : 1 : 1 ratio to Compound A 50 mg BID, 100 mg BID, 200 mg QD, and placebo groups, with 20 subjects per group.

The core treatment period lasted 12 weeks in total. Subjects returned to the site for clinical visits every 4 weeks during the study period. Data cleaning, locking, and analysis were performed after the last enrolled subject completed the clinical visit at week 12.
- Extended treatment period:
   The extended treatment period lasted 12 weeks in total. Subjects returned to the site for clinical visits every 4 weeks during the treatment period.
- Safety follow-up period:
   Subjects returned to the site for a safety follow-up visit 4 weeks after the last dose of medication. Subjects were discontinued from the study after completing the protocol-specified safety indicator collection and assessments. Any adverse event (AE) will be followed up in accordance with the provisions in Section 7 of the protocol.

### 3. Study population

Enrollment of eligible subjects was critical for the study to achieve the intended objectives. All medical or non-medical conditions of each subject were taken into consideration when determining their eligibility for the trial.

### Run-in period inclusion criteria (subjects must meet all of the following criteria to enter the run-in period)

1) prior to any study-related activities, being able to understand the nature, objectives, procedures and requirements of this study, as well as potential risks, volunteering to participate in this trial, and providing written informed consent forms;
2) age ≥ 18 years (at the time of signing informed consent forms), male or female;
3) body weight ≥ 35 kg and BMI < 35 kg/m²;
4) confirmed diagnosis of primary IgA nephropathy by renal pathological biopsy within 5 years prior to screening, with no known secondary causes (including but not limited to anaphylactoid purpura, systemic lupus erythematosus, etc.);
5) 24h-UPE ≥ 0.75 g/24h, or FMV UPCR ≥ 0.8 g/g at screening;
6) eGFR ≥ 30 ml/min/1.73m² at screening; (calculated using the CKD-EPI2021 equation)
7) laboratory tests at screening meeting the following criteria:
   a) AST and ALT ≤ 3.0 × ULN; total bilirubin ≤ 2.0 × ULN;
   b) hemoglobin ≥ 90 g/L;
   c) platelet count ≥ 80 × 10⁹/L;
   d) female subjects must meet either of the following criteria:
      a) being non-fertile, i.e., surgically sterilized (hysterectomy, bilateral salpingectomy, or bilateral oophorectomy performed at least 6 weeks prior to screening) or postmenopausal (defined as 12 consecutive months of amenorrhea with no other medical cause), or
      b) if of childbearing potential, she must have a negative pregnancy test at screening; and she must agree not to attempt conception, not to donate ova, and to use an effective contraceptive method from the time of signing the informed consent form until at least 30 days after the last dose of investigational medicinal product;
8) male subjects not surgically sterilized must agree not to donate sperm from the time of signing the informed consent form until at least 90 days after the last dose of study drug; if engaging in sexual intercourse with a female partner of childbearing potential, the male subject must voluntarily use a condom in conjunction with an effective contraceptive method used by the female partner.

### Randomization inclusion criteria (subjects must meet all of the following criteria to undergo randomization)

9) for subjects who have received the optimized RAS blocker (ACEI or ARB) therapy for ≥ 12 weeks prior to randomization, with a stable dose for ≥ 4 weeks after reaching the maximum recommended dose or maximum tolerated dose, those judged by the investigator to be intolerant to RAS blockers can be enrolled.
10) subjects who have received SGLT2 inhibitors, endothelin receptor antagonists, or hydroxychloroquine must have been on stable therapy for ≥ 12 weeks prior to randomization, with the prescription expected to remain stable throughout the study period;
11) 24h-UPE ≥ 0.75 g/24h prior to randomization;
12) eGFR ≥ 30 ml/min/1.73m² prior to randomization; (calculated using the CKD-EPI2021 equation)
13) vaccination against *Neisseria meningitidis* and *Streptococcus pneumoniae* at least 2 weeks should be performed before the first dose of Compound A tablets; if Compound A tablets treatment must start fewer than 2 weeks after vaccination, prophylactic antibiotic therapy must be initiated and continued until at least 2 weeks post vaccination.

### Subjects who met any of the following criteria were ineligible for enrollment in this study:

1) subjects who have participated in any clinical trial of drugs or medical devices within 3 months prior to screening, with residual effects of the trial treatment anticipated (as judged by the investigator), or have been within the follow-up period of another clinical trial or within 5 half-lives of the investigational drug (whichever is longer) at screening;
2) known or suspected immunodeficiency diseases (as judged by the investigator) or suffering from hereditary complement deficiency;
3) uncontrolled blood pressure at screening as assessed by the investigator (e.g., systolic blood pressure ≥ 160 mmHg or diastolic blood pressure ≥ 100 mmHg);
4) uncontrolled blood glucose at screening as assessed by the investigator (e.g., fasting blood glucose ≥ 11.1 mmol/L);
5) rapidly progressive glomerulonephritis (eGFR decline > 50% within 3 months, or < 50% but at risk of rapid renal function deterioration as assessed by the investigator); renal pathology indicating the formation of crescents (referring to large crescents, i.e., crescents occupying not less than 50% of the volume of Bowman's capsule) in not less than 50% of the glomeruli; tubular atrophy-interstitial fibrosis > 50%;
6) a history of bone marrow/hematopoietic stem cell transplantation or solid organ transplantation;
7) diagnosis of malignancy within the past 5 years (excluding radically resected basal cell carcinoma of the skin, papillary thyroid carcinoma, squamous cell carcinoma of the skin, or cervical carcinoma in situ);
8) a previous history of infection caused by encapsulated bacteria (e.g., *Neisseria meningitidis*, *Streptococcus pneumoniae*, *Haemophilus influenzae*, etc.);
9) past or current diagnosis of active tuberculosis, or untreated latent tuberculosis;
10) history or signs of any significant chronic active or recurrent infection within 6 months prior to screening, or laboratory evidence at screening of a significant chronic active or recurrent infection requiring antibacterial, antiviral, or antifungal therapy;
11) human immunodeficiency virus (HIV) infection (known HIV history or positive HIV antibody screening), active syphilis infection; active hepatitis B virus infection (positive for hepatitis B surface antigen and HBV DNA ≥ 1 × ULN), hepatitis C virus infection (positive for HCV antibody and HCV RNA ≥ 1 × ULN);
12) subjects who have received systemic glucocorticoid therapy (prednisone or equivalent, including budesonide enteric capsules), immunosuppressants/immunomodulators (e.g., mycophenolic acid drugs, cyclosporine, calcineurin inhibitors, etc.), or Chinese patent medicines with immunosuppressive effects (*Tripterygium wilfordii* preparations, *Sinomenium acutum* preparations, etc.) within 12 weeks prior to dosing; or subjects who, in the investigator's judgment, may require the above treatment during the study period;
13) subjects who have received aldosterone receptor antagonists, renin inhibitors, or drugs that may significantly affect creatinine levels (e.g., Niaoduqing Granules, Shenshuaining, fibrate lipid-lowering agents, medicinal charcoal tablets, etc.) within 4 weeks prior to dosing; or subjects who, in the investigator's judgment, may require the above treatment during the study period;
14) subjects who have received treatment of biologics, such as anti-CD20 monoclonal antibodies, anti-CD38 monoclonal antibodies, or cytokine inhibitors; or other complement pathway inhibitors other than the investigational drug used in this study within 6 months prior to dosing;
15) significant comorbidities as judged by the investigator, including but not limited to advanced cardiac disease (e.g., New York Heart Association (NYHA) Classes III and IV), severe pulmonary disease (e.g., severe pulmonary hypertension (World Health Organization (WHO) Classes III and IV)), or severe hepatic disease;
16) subjects with a history of gastrointestinal surgery that may significantly affect drug absorption, distribution, metabolism, and excretion, or a history of severe gastrointestinal disease, or with dysphagia or recurrent vomiting resulting in difficulty eating or taking medication;
17) hypersensitivity to any component of the investigational medicinal product;
18) subjects with a history of blood donation or severe blood loss (blood volume ≥ 400 mL) within 12 weeks prior to screening, or blood transfusion within 12 weeks prior to screening;
19) subjects with a history of severe trauma or major surgery within 12 weeks prior to screening, or planning to undergo major surgery during the trial;
20) pregnant or lactating females, or females planning to become pregnant during the study and follow-up period; and
21) any medical condition that, in the investigator's judgment, may interfere with the subject's participation in the study, any condition that may expose the subject to significant safety risks or indicate poor compliance, and other conditions deemed inappropriate for study participation by the investigator.

### 4. Study intervention

In accordance with the definition of investigational medicinal product in the current Good Clinical Practice (referring to the investigational drug and comparator drug used in clinical trials), the investigational medicinal products in this study include: Compound A tablets and placebo.

### 4.1 Information of investigational medicinal products

**Investigational drug:** Compound A tablets, supplied by Sichuan Haisco Pharmaceutical Co., Ltd., and prepared according to the method recorded in WO 2024175028 A1; dosage form: tablet; specification: 25 mg and 100 mg; storage conditions: tightly closed storage; and shelf life: provisionally 24 months.

**Comparator drug (placebo):** Compound A tablet matching placebo. It had identical packaging, administration route, labeling, appearance, taste, odor, etc., as Compound A tablets to conceal the actual treatment allocation. All matching placebos were manufactured by Sichuan Haisco Pharmaceutical Co., Ltd. in accordance with the specification and appearance of Compound A tablets, with the storage conditions being consistent with those of study drug Compound A tablets.

### 4.2 Dosing regimen

### Dosage and administration:

Subjects who passed screening and met the randomization criteria were randomized in a 1:1:1:1 ratio to each investigational drug group and the placebo group. The specific dosing regimen was as follows:
the drugs were administered twice daily, in the morning and evening, either in a fasted state or after a meal, and taken with an adequate amount of water, and the treatment duration followed the requirements described in the section below.

The total treatment period in this study was 24 weeks, including a 12-week core treatment period and a 12-week extended treatment period.

**Table 7. Dosing mode**

| Treatment group | Treatment period | | |
|---|---|---|---|
| | Dose per administration | Administration method | Daily dose |
| Compound A 50 mg BID dose group | 50 mg | Compound A 25 mg × 2 tablets + 100 mg matching placebo × 2 tablets | 100 mg |
| | | Compound A 25 mg × 2 tablets + 100 mg matching placebo × 2 tablets | |
| Compound A 100 mg BID dose group | 100 mg | Compound A 100 mg × 1 tablet + 100 mg matching placebo × 1 tablet + 25 mg matching placebo × 2 tablets | 200 mg |
| | | Compound A 100 mg × 1 tablet + 100 mg matching placebo × 1 tablet + 25 mg matching placebo × 2 tablets | |
| Compound A 200 mg QD dose group | 200 mg | Compound A 100 mg × 2 tablets + 25 mg matching placebo × 2 tablets | 200 mg |
| | | 100 mg matching placebo × 2 tablets + 25 mg matching placebo × 2 tablets | |
| Placebo group | - | 100 mg matching placebo × 2 tablets + 25 mg matching placebo × 2 tablets | - |

### 5. Study procedures

Prior to initiating any study procedure, subjects were required to read and sign the ICF approved by the Ethics Committee. The subject's eligibility was confirmed by the investigator before enrollment. Detailed study flow and assessment schedules were provided in the trial flow charts (Tables 8 and 9). All study steps must be performed within the time windows specified in the trial flow charts (Tables 8 and 9). Visit timing and time windows were calculated with the date of the first dose as Day 1 (D1).

The study flow included a screening period, a run-in period, a core treatment period, an extended treatment period, and a safety follow-up period.

### End of study

Defined as the last subject completing the final safety follow-up visit.

### 6. Study evaluation

Every effort shall be made to ensure that all tests and procedures required by the protocol should be completed as planned. If a protocol-required test can not be performed, the investigator shall document the reason, state what corrective and preventive measures have been taken to keep the clinical trial on track in accordance with the protocol, and promptly notify the study team of such unexpected events.

### 6.1 Evaluation of efficacy

Subjects were evaluated for efficacy by collecting laboratory indicators for evaluating renal function at scheduled visit time points, including 24h-UPCR, 24h-UPE, as well as FMV UPCR, UACR, eGFR, serum creatinine, and the like. All the above-mentioned indicators were tested at the central laboratory at baseline and during the dosing period visits.

### 6.2 Evaluation of safety

Safety monitoring was performed via vital signs, physical examination, 12-lead ECG, laboratory tests, pregnancy testing, concomitant therapy, and collection of all AEs (including serious adverse events (SAEs) and monitored events).

### Adverse events

The evaluation included the type, severity, onset and end time, classification as a serious adverse event, relationship to the investigational medicinal product, outcome, and the like. AEs occurring during the study, including signs and symptoms during the screening period, were documented in the source medical records.

### 6.3 Evaluation of pharmacokinetics

PK blood samples were collected at the first dose on D1, and at V4, V5, V6, V8 and V9. Detailed collection time points was provided in the trial flow charts (Tables 8 and 9). PK blood samples were sent to a bioanalytical laboratory for determination of blood drug concentrations of Compound A.

### 6.4 Evaluation of pharmacodynamics

PD blood samples and/or urine samples were collected at the first dose on D1, and at V3, V4, V5, V6, V8 and V9. Detailed collection time points was provided in the trial flow charts (Tables 8 and 9). PD blood samples and urine samples were sent to a bioanalytical laboratory for testing.

### 7. Definitions of adverse events

**Adverse events (AE):** It refers to all adverse medical events appearing in a subject during a clinical study, which can be manifested as symptoms, signs, diseases, or abnormal laboratory test results, but may not necessarily have a causal relationship with the investigational medicinal product. AEs include, but are not limited to:
1) exacerbation of a pre-existing medical condition/disease (prior to clinical trial entry) (including exacerbation of symptoms, signs, abnormal laboratory test results);
2) any newly occurring AE: any newly occurring adverse medical condition (including symptoms, signs, newly diagnosed diseases);
3) clinically significant abnormal laboratory test results;
4) if a clearly abnormal laboratory test value was deemed by the investigator to have no clinical significance, the reason should be clearly documented in the source documents (e.g., normal fluctuation of a disease).

### 8. Indicator analysis

### 8.1 Efficacy analysis

### 8.1.1 The five attributes of the primary estimand

- Treatment: treatment with Compound A 50 mg BID, 100 mg BID, 200 mg QD, or placebo given on the basis of optimized supportive treatment;
- Population: patients with a diagnosis of primary IgA nephropathy confirmed by renal biopsy, who met the inclusion criteria, did not meet any exclusion criteria, and were randomized.
- Variable: ratio of 24h-UPCR after 12 weeks of treatment relative to baseline;
- Concomitant events and handling strategy: concomitant events included treatment discontinuation in advance, adjustments to background therapy during the treatment period, and administration of prohibited therapy during the treatment period, and the data collected after all concomitant events were included in statistical analysis based on the therapy strategy;
- Population-level summary: least squares geometric mean ratios between groups.

### 8.1.2 Primary efficacy endpoint analysis

The primary analysis of the study's primary estimand was based on FAS using a mixed-effects model for repeated measures (MMRM) to estimate the least squares means for the ratio of 24h-UPCR after 12 weeks of treatment in each treatment group relative to baseline and 95% CIs, as well as the least squares geometric mean ratios between each trial group and the placebo group and 95% CIs. The MMRM model included data from baseline through all scheduled visits up to week 12 of the treatment period (following natural log transformation), with randomization stratification factors, treatment group, visit and treatment-by-visit interaction as fixed effects, log-transformed baseline 24h-UPCR as a covariate, and subject as a random effect. An unstructured (UN) covariance matrix was used in the model to fit the correlations among repeated measurements of subjects (if the model failed to converge, covariance matrix structures such as TOEPH, ARH (1), or CS can considered in the order specified). Covariance matrix parameters were estimated using the restricted maximum likelihood (REML) method. Degrees of freedom were calculated using the Kenward-Roger method.

Furthermore, a dose-response relationship was explored for the primary estimand, and analysis was performed based on monotonic dose-response models including linear models, Emax model and the like.

### 8.1.3 Secondary endpoint analysis

Analysis of secondary efficacy indicators was based on FAS. For secondary efficacy endpoints (ratios of 24h-UPCR relative to baseline at other clinical visits during the treatment period, ratios of 24h-UPE relative to baseline at each clinical visit during the treatment period, and ratios of FMV UPCR and UACR relative to baseline at each clinical visit during the treatment period), analysis was performed using the MMRM model following natural log transformation. All scheduled visit data from baseline through week 24 of the treatment period were included, with randomization stratification factors, treatment group, visit, and treatment-by-visit interaction as fixed effects, log-transformed baseline value as a covariate, and subject as a random effect. For the changes relative to baseline in estimated glomerular filtration rate (eGFR) at each clinical visit during the treatment period and the changes relative to baseline in serum creatinine at each clinical visit during the treatment period, the MMRM model was used, with randomization stratification factors, treatment group, visit, and treatment-by-visit interaction as fixed effects, the log-transformed baseline value as a covariate, and subject as a random effect.

### 8.2 Safety analysis

Safety analysis was performed based on the Safety Analysis Set. Data were summarized and tabulated by phase and the actual treatment group subject received, including but not limited to statistical descriptions and tabulating of adverse events, laboratory data, vital sign data, electrocardiogram data, and the like of subjects.

### 8.3 Pharmacokinetic analysis

Statistical analysis of PK parameters was descriptively summarized based on the PK parameter analysis dataset. Non-compartmental model was used to estimate and analyze pharmacokinetic parameters. Primary pharmacokinetic parameters included, but were not limited to, AUC0-τ, Tmax, Cmax, t1/2, CL/F, Vd/F, and the like.

Statistical description was generated for blood drug concentrations of Compound A at the predefined blood sampling time points. Data from this study were pooled with data from other clinical trials of Compound A to establish a population pharmacokinetic model and evaluate the effects of internal and external covariates on the pharmacokinetic properties of Compound A. In addition, exposure-response analysis was performed for specific efficacy and safety endpoints. Results of the above population pharmacokinetic and exposure-response analysis were written in a separate report.

### 8.4 Pharmacodynamic analysis

Based on PDS, descriptive summaries and tabulations were performed for changes relative to baseline in AP activity, plasma Bb fragment levels, and sC5b-9 levels in plasma and urine.

### 9. Conclusions of the study

The results of this randomized, double-blind, placebo-controlled, parallel-group phase II clinical study indicate that Compound A tablets demonstrate favorable efficacy and safety in the treatment of primary IgA nephropathy.

## Claims

1. Use of a compound of formula (I) or a stereoisomer, tautomer, pharmaceutically acceptable salt, cocrystal or composition thereof in the preparation of a drug for preventing or treating a complement factor B-mediated disease or condition, **characterized in that** an effective dose of the compound of formula (I), or the stereoisomer, tautomer, pharmaceutically acceptable salt, cocrystal or composition thereof is administered to a mammal, 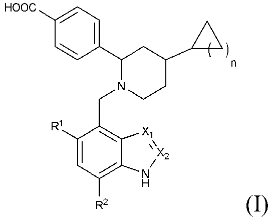 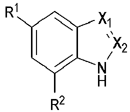 is selected from 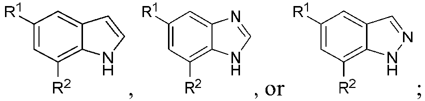
R¹ is selected from -OCH₃ or -OCD₃;
R² is selected from -CH₃ or -CD₃;
n is selected from 1, 2 or 3; and
the complement factor B-mediated disease or condition is selected from at least one of: paroxysmal nocturnal hemoglobinuria, primary glomerulonephritis, membranous nephropathy, C3 glomerulonephritis, or atypical hemolytic uremic syndrome.

2. The use according to claim 1, **characterized in that** the compound of formula (I) is selected from the following structures: 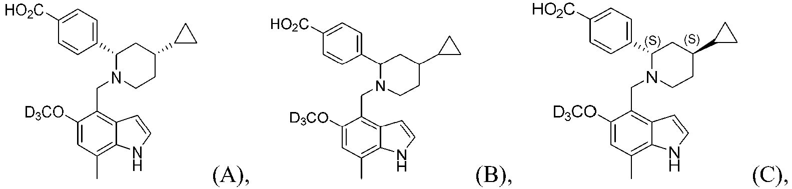 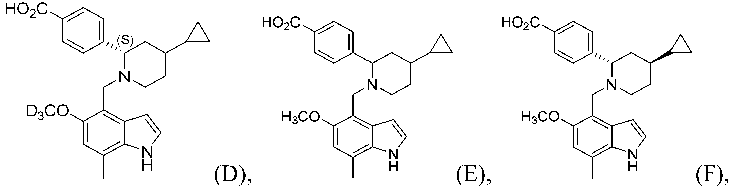 or

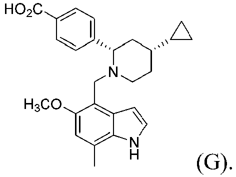

3. The use according to claim 2, **characterized in that** an effective dose of the compound of formula (A), or the stereoisomer, tautomer, pharmaceutically acceptable salt, cocrystal or composition thereof is administered to a mammal.

4. The use according to claim 3, **characterized in that** the pharmaceutically acceptable salt is selected from hydrochloride, succinate, sulfate, maleate, phosphate, tartrate, fumarate, citrate, naphthalene disulfonate, p-toluenesulfonate, methanesulfonate, benzenesulfonate, oxalate, gentisate, succinate, or hydrobromide.

5. The use according to any one of claims 1 to 4, **characterized in that** the effective dose is based on a free base of formula (A) and is 1-2400 mg/day.

6. The use according to claim 5, **characterized in that** the compound of formula (A), or the stereoisomer, tautomer, pharmaceutically acceptable salt, cocrystal or composition thereof is administered at an interval selected from once a day, twice a day, three times a day, once a week, twice a week, three times a week or once every other day.

7. The use according to claim 6, **characterized in that** the compound of formula (A), or the stereoisomer, tautomer, pharmaceutically acceptable salt, cocrystal or composition thereof is administered via a route selected from oral route, injection, drip, transdermal route, sublingual route, parenteral and intraperitoneal route, rectal route, buccal route, nasal spray, nasal drip, inhalation, local delivery, subcutaneous route, intra-adipose route, intra-articular route, intraperitoneal route or intrathecal route, preferably oral route, injection and nasal spray.

8. The use according to claim 7, **characterized in that** the compound of formula (A), or the stereoisomer, tautomer, pharmaceutically acceptable salt, cocrystal or composition thereof is administered via a route selected from oral route, intravenous injection, intravenous drip, intra-arterial injection, intramuscular injection, subcutaneous injection, intra-articular injection, intraperitoneal injection, intrathecal injection or nasal drip.

9. A method for treating a complement factor B-mediated disease or condition, **characterized in that** the method comprises administering an effective dose of the compound of formula (I) or formula (A), or the stereoisomer, tautomer, pharmaceutically acceptable salt, cocrystal or composition thereof as defined in any one of claims 1-4 to a mammal, the effective dose being 1-2400 mg/day, preferably 20 mg-50 mg/day, 60 mg-150 mg/day, 120 mg-300 mg/day, 240 mg-600 mg/day, 320 mg-800 mg/day, 40 mg-100 mg/day, 120 mg-300 mg/day, 240 mg-600 mg/day, 480 mg-1200 mg/day, 640-1600 mg/day, 60 mg-150 mg/day, 180 mg-450 mg/day, 360 mg-9000 mg/day, 720 mg-1800 mg/day, or 96-2400 mg/day;
the complement factor B-mediated disease or condition is selected from at least one of: paroxysmal nocturnal hemoglobinuria, primary glomerulonephritis, membranous nephropathy, C3 glomerulonephritis, or atypical hemolytic uremic syndrome.

10. The method according to claim 9, **characterized in that** the compound of structural formula (A), or the stereoisomer, tautomer, pharmaceutically acceptable salt, cocrystal or composition thereof is administered at an interval selected from once a day, twice a day, three times a day, once a week, twice a week, three times a week or once every other day.

11. The method according to claim 9 or 10, **characterized in that** the compound of formula (A), or the stereoisomer, tautomer, pharmaceutically acceptable salt, cocrystal or composition thereof is administered via a route selected from oral route, injection, drip, transdermal route, sublingual route, parenteral and intraperitoneal route, rectal route, buccal route, nasal drip, inhalation, local delivery, subcutaneous route, intra-adipose route, intra-articular route, intraperitoneal route or intrathecal route.

12. The method according to claim 11, **characterized in that** the compound of formula (A), or the stereoisomer, tautomer, pharmaceutically acceptable salt, cocrystal or composition thereof is administered via a route selected from oral route, intravenous injection, intravenous drip, intra-arterial injection, intramuscular injection, subcutaneous injection, intra-articular injection, intraperitoneal injection, intrathecal injection or nasal drip.
